Europäisches Patentamt

(19) European Patent Office

Office européen des brevets

(11) Numéro de publication: **0 243 238 B1**

(12) **FASCICULE DE BREVET EUROPEEN**

(45) Date de publication du fascicule du brevet: **08.11.89**

(21) Numéro de dépôt: **87400836.0**

(22) Date de dépôt: **14.04.87**

(51) Int. Cl.⁴: **C07C 7/163, B01J 23/88**

---

(54) Procédé et catalyseur de desulfuration des essences terpeniques.

---

(30) Priorité: **18.04.86 FR 8605572**

(43) Date de publication de la demande:
**28.10.87 Bulletin 87/44**

(45) Mention de la délivrance du brevet:
**08.11.89 Bulletin 89/45**

(84) Etats contractants désignés:
**AT ES SE**

(56) Documents cités:
**US-A- 3 312 750**

(73) Titulaire: **SOCIETE NATIONALE ELF AQUITAINE (PRODUCTION), Tour Elf 2, Place de la Coupole La Défense 6, F-92400 Courbevoie(FR)**
Titulaire: **SOCIETE DES DERIVES RESINIQUES ET TERPENIQUES, 30 rue Gambetta, F-40300 Dax(FR)**

(72) Inventeur: **Casbas, Françoise, 1 rue Saint-Pierre, F-64300 Orthez(FR)**
Inventeur: **Duprez, Daniel, 21 Bis rue du Touffenet, F-86000 Poitiers(FR)**
Inventeur: **Ollivier, Jean, Croix de Buzy, F-64260 Arudy(FR)**
Inventeur: **Rolley, Raymond, "Paile", F-40560 Vielle Saint-Girons(FR)**

(74) Mandataire: **Kohn, Armand, 5 Avenue Foch, F-92380 Garches(FR)**

---

## Description

La présente invention se rapporte à la purification des essences terpéniques renfermant des impuretés sulfurées ; elle s'applique en particulier aux essences qui constituent un sous-produit de la fabrication du papier. L'invention concerne plus spécialement un procédé de purification résidant dans la désulfuration catalytique, par de l'hydrogène, de coupes terpéniques issues de la distillation des essences brutes, impures.

L'industrie papetière est un fournisseur important d'essence de papeterie qui contient surtout les α-pinène , β-pinène, Δ³carène, myrcène, dipentène et éventuellement des dérivés moins fréquents, comme camphène et autres. Malheureusement, le procédé de délignification du bois, utilisé pour libérer la cellulose, dit procédé KRAFT, basé sur la solubilisation de la lignine à l'aide d'un mélange de soude et de sulfure de sodium, introduit des quantités notables de polluants soufrés dans l'essence extraite des autoclaves de cuisson de la pâte de bois. Ces polluants sont une gêne importante par l'odeur désagréable qu'ils introduisent d'une part, et par les modifications chimiques et physiques des terpènes d'autre part. Il faut donc éliminer ces composés soufrés, afin de retrouver les propriétés des terpènes issus de l'essence de térébenthine dite de gemme. De nombreuses méthodes ont pour cela été mises au point. Les plus utilisées sont basées sur le traitement au chlorite de sodium,à l'hypochlorite de sodium ou à l'éthylène-diamine. Mais ces traitements présentent plusieurs inconvénients parmi lesquels : opérations lourdes, nécessitant un grand nombre d'étapes qui rendent difficiles le traitement continu ; ces traitements chimiques classiques introduisent dans les terpènes des produits indésirables contenant du chlore, ou de l'azote ; ces procédés désodorisent, mais ne désulfurent pas vraiment, leur efficacité restant limitée.

Pour y remédier on a proposé, en 1963, un procédé de désulfuration de l' α-pinène par un traitement catalytique à l'hydrogène. Ainsi, le brevet US 3 312 750 indique-t-il le passage de l' α-pinène en contact avec un catalyseur de molybdate de cobalt sur de l'alumine, à une température de 149° à 260°c, sous une pression d'hydrogène de 0 à 35 bars. Ce procédé s'est révélé bien efficace en ce qu'il permet l'élimination d'environ 66 à 90% du soufre présent dans les terpènes, mais il comporte l'inconvénient de transformer une forte fraction de l' α-pinène ; cette altération chimique est due en particulier à l'isomérisation et à la saturation des doubles liaisons des composés traités. Elle conduit à des composés beaucoup moins intéressants pour l'industrie que ne le sont les composants de l'essence de térébenthine non modifiée.

Cette question de transformation est mentionnée dans le brevet US précité, uniquement dans le cas d'expérimentation à hautes pressions, mais - lorsqu'on cherche à reproduire ces opérations - on constate une transformation des produits traités atteignant jusqu'à 55% environ.

La présente invention remédie aux inconvénients susindiqués du procédé d'hydrodésulfuration catalytique, décrit dans le brevet US 3 312 750. Elle permet d'effectuer la désulfuration aussi efficacement, de l' α-pinène ainsi que d'autres coupes terpéniques, sans toutefois modifier sensiblement la composition chimique du terpène traité.

En effet, alors que le procédé selon l'invention peut conduire à une élimination d'environ 90% du soufre présent dans l'essence brute de papeterie, il n'entraîne qu'une faible transformation, inférieure à 8%, de l' α-pinène, contre environ 42% dans le cas du brevet US mentionné plus haut.

Le procédé de l'invention résulte de la constatation tout à fait inattendue que le remplacement du support d'alumine du catalyseur d'oxydes de Co et Mo par du charbon actif, présentant certaines caractéristiques spécifiques, se traduit par une diminution considérable de la tendance à la transformation chimique des terpènes, alors que l'activité de désulfuration reste excellente. En fait, rien ne pouvait laisser prévoir un tel changement de l'activité du catalyseur. Le résultat suivant l'invention est d'autant plus surprenant que la transformation des terpènes, sous l'effet du catalyseur selon le brevet US, reste forte même lorsqu'on traite préalablement l'alumine par de la soude pour supprimer les sites susceptibles de contribuer à l'isomérisation.

Ainsi, le procédé suivant l'invention, qui consiste à faire passer la vapeur des terpènes à désulfurer, avec de l'hydrogène, sur un catalyseur d'oxydes de Co et Mo, est caractérisé en ce que ce catalyseur est supporté par du charbon actif.

Conformément à l'invention,le charbon actif employé doit présenter une surface spécifique d'au moins 500 m²/g, en particulier 500 à 1500 m²/g et de préférence 700 à 1100 m²/g. Les diamètres des pores de ce support doivent être de l'ordre de 0,002 à 0,01, et surtout de 0,004 à 0,006 microns. De préférence,le charbon actif utilisé est exempt de tout composé du silicium.

Au moment de sa mise en service, le catalyseur suivant l'invention titre en général 3 à 10% en poids de CoO et 3 à 15% de MoO₃, le rapport molaire de CoO/MoO₃ pouvant varier entre 1/5 et 5. Cependant, alors que selon la technique antérieure le rapport préféré était de 1 CoO pour 2,6 MoO₃, le procédé de l'invention donne des résultats meilleurs avec, au contraire, un excès de cobalt par rapport au molybdène, notamment 2 à 5 CoO, et surtout 2,5 à 3,5, pour 1 MoO₃. Il en résulte que les proportions pondérales, dans le nouveau catalyseur, sont de préférence 5 à 9% CoO avec respectivement 2,77 à 5,66% MoO₃, la différence à 100 étant constituée par le charbon actif.

La préparation du catalyseur s'effectue à la manière connue dansl'art. Le charbon actif est imprégné d'une solution aqueuse de sel thermodissociable de chacun des métaux Co et Mo, par exemple nitrate de Co et molybdate d'ammonium. Le mélange est séché, puis calciné à une température de 450°C à 550°C.

L'hydrodésulfuration des terpènes, avec le catalyseur suivant l'invention, est de préférence effectuée à une température de 160° à 300°C, et surtout entre 180° et 220°C, sous une pression relative d'hydrogène de 0 à 5 bars et avec une vitesse spatiale du réactif comprise entre 0,1 et 10 h⁻¹. Les rapports molaires préférés H₂/terpènes se rangent entre 1 et 15. Les conditions particulièrement favorables sont : température 200° ± 5°C ; pression 0,1 à 1 bar ; vitesse spatiale 0,2 à 0,5 h⁻¹ ; rapport H₂/terpènes 5 à 8.

L'invention est illustrée par les exemples qui suivent.

### EXEMPLE I

Essai d'un catalyseur commercial de la technique antérieure à l'HDS d'une coupe terpénique de Δ³-carène, provenant de la distillation d'une essence de papeterie.

Le catalyseur utilisé contient approximativement, en poids, 5% CoO et 13% MoO₃.

La coupe terpénique traitée est du Δ³-carène dont la teneur en soufre est de 0,12%.

Elle est soumise au chauffage au contact du catalyseur susindiqué, dans les conditions suivantes : Température 200°C, sous la pression atmosphérique ; vitesse spatiale de passage du réactif 0,86 h⁻¹ ; rapport molaire hydrogène/terpène = 7 ; volume de catalyseur : 70 ml ; débit de l'hydrogène 1 l/h et par ml de terpène dans les conditions normales de température et de pression. On arrive ainsi à n'avoir plus que 0,03% de soufre dans le Δ³-carène traité, ce qui représente une élimination de 72% du soufre initial. Cependant, 54% du Δ³-carène de départ ont été transformés.

### EXEMPLE 2

Le catalyseur utilisé est préparé par l'imprégnation de billes d'une alumine de surface spécifique de 80 m²/g, présentant un volume poreux de 0,65 ml/g, avec des solutions aqueuses de CoNO₃ et Na₂MoO₄. Après séchage de 2 h à 150°C et calcination, le catalyseur contient, en poids, 7% CoO et 4,4% MoO₃.

On opère à 200°C, sous la pression atmosphérique, avec un volume de catalyseur de 150 ml et une vitesse spatiale du réactif de 0,21 h⁻¹, le rapport molaire H₂/terpène est toujours 7.

La coupe terpénique traité est du Δ³-carène.

Le taux initial de soufre passe de 1290 ppm à 136 ppm ce qui représente une élimination de 89,4%, mais le taux de transformation est de 5,5%.

### EXEMPLE 3

Le catalyseur utilisé est toujours celui de l'exemple 2. Il sert à traiter une coupe terpénique d' α-pinène. Après 23 heures de temps de fonctionnement, le taux de soufre passe de 700 à 224 ppm, ce qui correspond à une élimination de 68%. Le taux de transformation de l'α-pinène est de 25%.

### EXEMPLES 4 à 6

Hydrodésulfuration des terpènes sur le catalyseur supporté par du charbon actif

### Exemple 4

Charbon actif seul, sans Co ni Mo

Afin de connaître le rôle du charbon actif lui-même, on effectue d'abord une hydrodésulfuration avec des grains d'un charbon actif présentant une surface spécifique de 900 m²/g et un diamètre des pores de 0,005 microns, pour traiter une coupe terpénique de Δ³-carène. Dans les mêmes conditions opératoires que pour l'exemple 2, le taux initial de soufre, de 2760 ppm, passe à 960 ppm, soit une élimination de 65,2%; le taux de transformation du Δ³-carène est de 0,5%. Ainsi voit-on que le charbon actif seul n'a qu'une très faible action modificatrice du terpène.

### Exemple 5

Catalyseur suivant l'invention

Le charbon, utilisé dans l'exemple 4, joue ici le rôle de support pour l'imprégnation des sels de cobalt et de molybdène.

Le catalyseur, préparé comme indiqué à l'exemple 1 (sauf nature du support), contient 7% CoO et

3

4,4% MoO₃, en poids. Il sert à traiter à l'hydrogène une coupe terpénique d' α-pinène dans les conditions opératoires suivante : T : 200°C. pression atmosphérique ; vitesse spatiale 0,2 h⁻¹ ; rapport molaire H₂/terpène = 7 ; volume de catalyseur 140 ml ; débit de l'hydrogène 30 l/h ; débit de terpène 30 ml/h.

Le taux de soufre passe de 88 ppm à 10 ppm, soit une élimination de 88,6%. Le taux de transformation n'est que de 7,3%.

### Exemple 6

Le même catalyseur, que dans l'exemple 5, est utilisé pour traiter une coupe terpénique de $\Delta^3$-carène. Le taux de soufre initial de 1200 ppm passe à 360 ppm. Le taux de transformation du $\Delta^3$-carène est de 1%. On voit que le catalyseur suivant l'invention produit une élimination de 70% de S et transforme seulement 1% de $\Delta^3$-carène contre 54% dans le procédé de l'art antérieur.

### Récapitulation comparative des exemples

Dans le tableau qui suit sont réunis les résultats des exemples 1 à 6 à côté des principales données du brevet US 3 312 750.

| Exemple no | Désignation | | % S éliminé | % terpènes transformés |
|---|---|---|---|---|
| I | USP 3 312 750, α-pinène | | | |
| | CoMo sur alumine | # Oregon | 90,9 | non indiqué |
| | | # Washington | 66,6 | |
| 1 | CoMo sur alumine (commercial) $\Delta^3$-carène | | 72% | 54% |
| 2 | CoMo sur alumine (préparé) $\Delta^3$-carène | | 89,4% | 5,5% |
| 3 | α-pinène | | 68,0% | 25% |
| 4 | Charbon actif sans Co ni Mo $\Delta^3$-carène | | 65,2% | 0,5% |
| 5 | Nouveau catalyseur, CoMo sur charbon actif α-pinène | | 88,6% | 7,3% |
| 6 | Nouveau catalyseur $\Delta^3$-carène | | 70% | 1% |

La comparaison des exemples 1 avec 6 montre que le nouveau catalyseur (6), suivant l'invention, permet une élimination du soufre au moins aussi poussée que le catalyseur (1) de la technique antérieure ; de plus, ce nouveau catalyseur ne donne lieu qu'à une transformation des terpènes de 1% contre 54% dans le cas du catalyseur (1) sur support d'alumine. Il y a là un progrès très marqué.

Le % d'élimination du soufre, dans l'exemple 1, cadre bien avec la moyenne des éliminations selon exemple I du brevet US ; mais l'exemple 1 apporte un renseignement qui manque dans ce brevet, le taux de transformation de 54% des terpènes traités.

### Revendications

1. Procédé d'élimination du soufre des terpènes provenant de la fabrication du papier, par leur traitement en phase vapeur par de l'hydrogène, en présence d'un catalyseur à base d'oxydes de Co et de Mo, caractérisé en ce que ce catalyseur est utilisé sur un support de charbon actif.

2. Procédé suivant la revendication 1, caractérisé en ce que le charbon actif présente une surface spécifique d'au moins 500 m²/g, notamment de 500 à 1500 m²/g, et de préférence de 700 à 1100 m²/g.

3. Procédé suivant la revendication 2, caractérisé en ce que le diamètre des pores du charbon actif employé est de l'ordre de 0,002 à 0,01, et de préférence de 0,004 à 0,006 microns.

4. Procédé suivant une des revendications 1 à 3, caractérisé en ce que le charbon actif est exempt de tout composé du silicium.

5. Procédé suivant une des revendications précédentes, caractérisé en ce que le catalyseur, au moment de sa mise en service, contient en poids 3 à 10% de CoO et 3 à 15% de MoO₃ avec un rapport CoO/MoO₃ de 0,2 à 5, le reste étant le charbon actif.

6. Procédé suivant la revendication 5, caractérisé en ce que le catalyseur contient un excès de CoO par rapport à MoO₃, notamment 2 à 5 moles de CoO par 1MoO₃.

7. Procédé suivant la revendication 6, caractérisé en ce que le catalyseur contient en poids 5 à 9% de CoO avec respectivement 2,77 à 5,66% MoO₃, le reste étant du charbon actif.

8. Procédé suivant une des revendications 1 à 7, réalisé à une température de 160° à 300°C, de préférence entre 180° et 220°C, sous une pression d'hydrogène relative à 0 à 5 bars, avec une vitesse spatiale du mélange hydrogène + vapeur des terpènes de 0,1 à 10 h⁻¹, le rapport molaire de $H_2$/terpènes étant de 1 à 15.

9. Catalyseur à base de Co et Mo, pour l'hydrodésulfuration des terpènes issus de la fabrication du papier, caractérisé en ce que des oxydes de Co et de Mo sont fixés sur un support de charbon actif.

10. Catalyseur suivant la revendication 9, caractérisé en ce qu'il contient 5 à 9% en poids de CoO, et du $MoO_3$ à raison de 1 mole $MoO_3$ pour 2 à 5 moles CoO.

## Patentansprüche

1. Verfahren zur Eliminierung von Schwefel aus Terpenen, die aus der Papierherstellung herrühren, durch ihre Behandlung in der Dampfphase mit Wasserstoff in Gegenwart eines Katalysators auf Basis der Oxide von Co und Mo, dadurch gekennzeichnet, daß der Katalysator auf einem Aktivkohleträger verwandt wird.

2. Verfahren nach Anspruch 1, dadurch gekennzeichnet, daß die Aktivkohle eine spezifische Oberfläche von wenigstens 500 m²/g, insbesondere von 500 bis 1500 m²/g und vorzugsweise von 700 bis 1100 m²/g aufweist.

3. Verfahren nach Anspruch 2, dadurch gekennzeichnet, daß der Porendurchmesser der verwandten Aktivkohle in der Größenordnung von 0,002 bis 0,01 und vorzugsweise von 0,004 bis 0,006 Mikron liegt.

4. Verfahren nach einem der Ansprüche 1 bis 3, dadurch gekennzeichnet, daß die Aktivkohle frei von jeder Siliciumverbindung ist.

5. Verfahren nach einem der vorstehenden Ansprüche, dadurch gekennzeichnet, daß der Katalysator zum Zeitpunkt der Inbetriebnahme 3 bis 10 Gew.-% CoO und 3 bis 15 Gew.-% $MoO_3$ mit einem Verhältnis CoO/$MoO_3$ von 0,2 bis 5 enthält, wobei der Rest Aktivkohle ist.

6. Verfahren nach Anspruch 5, dadurch gekennzeichnet, daß der Katalysator einen Überschuß an CoO im Verhältnis zu $MoO_3$, insbesondere 2 bis 5 Mol CoO auf ein $MoO_3$, enthält.

7. Verfahren nach Anspruch 6, dadurch gekennzeichnet, daß der Katalysator 5 bis 9 Gew.-% CoO mit jeweils 2,77 bis 5,66 Gew.-% $MoO_3$ enthält, wobei der Rest Aktivkohle ist.

8. Verfahren nach einem der Ansprüche 1 bis 7, durchgeführt bei einer Temperatur von 160° bis 300°C, vorzugsweise zwischen 180° und 220°C, unter einem relativen Wasserstoffdruck von 0 bis 5 bar mit einer Raumgeschwindigkeit der Mischung aus Wasserstoff und Terpendampf von 0,1 bis 10 h⁻¹, wobei das molare Verhältnis von $H_2$/Terpenen 1 bis 15 ist.

9. Katalysator auf Basis von Co und Mo für die Hydroentschwefelung von Terpenen aus der Papierherstellung, dadurch gekennzeichnet, daß die Oxide von Co und Mo auf einem Aktivkohleträger fixiert sind.

10. Katalysator nach Anspruch 9, dadurch gekennzeichnet, daß er 5 bis 9 Gew.-% CoO und $MoO_3$ in einem Verhältnis von 1 Mol $MoO_3$ auf 2 bis 5 Mol CoO enthält.

## Claims

1. Process of elimination of sulphur from terpenes derived from the manufacture of paper, by their vapour phase treatment by hydrogen, in the presence of a catalyst based on oxides of Co and Mo, characterised in that the catalyst is used upon an active carbon substrate.

2. Process according to claim 1, characterised in that the active carbon has a specific surface of at least 500 m²/g, notably from 500 to 1500 m²/g and preferably from 700 to 1100 m²/g.

3. Process according to claim 2, characterised in that the diameter of the pores of the active carbon employed is of the order of 0.002 to 0.01 and preferably from 0.004 to 0.006 microns.

4. Process according to any of claims 1 to 3, characterised in that the active carbon is free from any silicon compound.

5. Process according to any of the preceding claims, characterised in that the catalyst at the time it is put to use contains by weight 3 to 10% of CoO and 3 to 15% of $MoO_3$ with a CoO/$MoO_3$ ratio of 0.2 to 5, the remainder being active carbon.

6. Process according to claim 5, characterised in that the catalyst contains an excess of CoO with respect of $MoO_3$, particularly 2 to 5 moles of CoO per 1$MoO_3$.

7. Process according to claim 6, characterised in that the catalyst contains by weight 5 to 9% of CoO with respectively 2.77 to 5.66% $MoO_3$, the remainder being active carbon.

8. Process according to any of claims 1 to 7, carried out at a temperature of 160° to 300°C, preferably between 180° and 220°C, under a relative hydrogen pressure of 0 to 5 bars, with a spatial velocity for the hydrogen + terpene vapour mixture of 0.1 to 10 h⁻¹, the molar ratio of $H_2$/terpenes being from 1 to 15.

9. Catalyst based on Co and Mo for the hydrodesulphurisation of terpenes derived from the manufacture of paper, characterised in that oxides of Co and of Mo are fixed to an active carbon substrate.

10. Catalyst according to claim 9, characterised in that it contains 5 to 9% by weight of CoO and $MoO_3$ in the ratio of 1 mole $MoO_3$ per 2 to 5 moles CoO.